(19) Europäisches Patentamt
European Patent Office
Office européen des brevets

(11) **EP 3 467 081 A1**

(12) **EUROPEAN PATENT APPLICATION**
published in accordance with Art. 153(4) EPC

(43) Date of publication:
**10.04.2019 Bulletin 2019/15**

(51) Int Cl.:
*C11D 1/14* *(2006.01)*          *C07C 43/10* *(2006.01)*
*C07C 43/11* *(2006.01)*        *C07C 229/16* *(2006.01)*
*C07C 309/20* *(2006.01)*       *C11D 1/72* *(2006.01)*
*C11D 1/74* *(2006.01)*         *D06L 1/12* *(2006.01)*

(21) Application number: **17806670.0**

(22) Date of filing: **30.05.2017**

(86) International application number:
**PCT/JP2017/020060**

(87) International publication number:
**WO 2017/209117 (07.12.2017 Gazette 2017/49)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **31.05.2016   JP 2016108442**

(71) Applicant: **Kao Corporation**
**Chuo-Ku**
**Tokyo 103-8210 (JP)**

(72) Inventors:
• **MORIKAWA Satoshi**
  **Wakayama-shi**
  **Wakayama 640-8580 (JP)**
• **INOUE Mayuko**
  **Tokyo 131-8501 (JP)**
• **KUSUNOKI Ayako**
  **Wakayama-shi**
  **Wakayama 640-8580 (JP)**
• **TSUMURA Kana**
  **Wakayama-shi**
  **Wakayama 640-8580 (JP)**

(74) Representative: **Hoffmann Eitle**
**Patent- und Rechtsanwälte PartmbB**
**Arabellastraße 30**
**81925 München (DE)**

(54) **DETERGENT COMPOSITION FOR FIBRES**

(57)     The present invention relates to a detergent composition for fibers containing the following component (A), component (B) and component (C):
component (A): an internal olefin sulfonate having 17 or more and 24 or less carbon atoms,
component (B): a nonionic surfactant, and
component (C): a metal ion chelating agent,
wherein the mass ratio (B)/(A) of component (B) to component (A) is 0 or more and 1.0 or less, the composition contains a nonionic surfactant having an HLB of more than 10.5 when the composition contains component (B), the content of component (C) is 20% by mass or less, and the composition is a composition for use in washing fibers in water containing a hardness component.

**EP 3 467 081 A1**

**Description**

Field of the Invention

The present invention relates to a detergent composition for fibers and a method for washing fibers.

Background of the Invention

Heretofore, anionic surfactants, particularly alkylbenzene sulfonates, have been widely used as household and industrial detergent components, because they have an excellent detergency and foaming capacity. As one of anionic surfactants other than alkylbenzene sulfonates, olefin sulfonates have been reported, particularly internal olefin sulfonates obtained by using, as a raw material internal, olefins having a double bond not at the end of an olefin chain but inside the olefin chain. It has been also known to use metal ion chelating agents such as zeolite in order to improve the detergency of anionic surfactants by capturing hardness components in washing water. It is further known that detergents contain nonionic surfactants having a polyoxyalkylene group in order to improve the detergency against stains attached to fibers.
JP-A 2014-76988 discloses an internal olefin sulfonate composition containing (A) an internal olefin sulfonate having 16 carbon atoms and/or (B) an internal olefin sulfonate having 18 carbon atoms wherein the mass ratio (A/B) of component (A) to component (B) contained in the composition is 0/100 to 70/30, which is especially excellent in foamability, foam quality, rapidly foaming capacity and defoaming property, and particularly foam quality when washing hair therewith.
EP-A 377261 discloses a detergent composition containing an internal olefin sulfonate, in which its β-hydroxy form is 25% or more, having an excellent detergent property. It describes that the internal olefin sulfonate has preferably 12 to 20 carbon atoms. Specifically, a granular laundry detergent composition containing an internal olefin sulfonate having 18 carbon atoms and 25% by mass of zeolite A is described.
JP-A 2003-81935 discloses a detergent composition containing an internal olefin sulfonate characterized in that it is obtained by sulfonating, neutralizing and hydrolyzing an internal olefin having 8 to 30 carbon atoms in which the total percentage of double bonds present at position 2 is 20 to 95% and the cis/trans ratio is 1/9 to 6/4. In Formulation Example 1, a granular detergent composition for clothing containing an internal olefin sulfonate, a nonionic surfactant having a polyoxyethylene group and 25% by mass of zeolite is described.
JP-A 3-126793 discloses a detergent composition containing an internal olefin sulfonate derived from an internal olefin having 12 to 18 carbon atoms and a nonionic surfactant having an HLB value of 10.5 or less in a particular ratio. In Examples, using an internal olefin sulfonate having 18 carbon atoms as a Comparative Example, the evaluation of washing with water the ionic strength of which has been adjusted with sodium chloride and sodium carbonate is described.

Summary of the Invention

However, the problem of softening fibers while maintaining detergency against stains attached to fibers is not described in any of JP-A 2014-76988, EP-A 377261, JP-A 2003-81935 and JP-A 3-126793. JP-A 3-126793 discloses a technique for enhancing detergency by using a nonionic surfactant having a low HLB and an internal olefin sulfonate in combination, but the present inventors have found that the nonionic surfactant having a low HLB inhibits the effect of softening fibers by an internal olefin sulfonate having a particular number of carbon atoms.
The present invention relates to a detergent composition for fibers and a method for washing fibers, which can finish fibers softly while maintaining detergency against stains attached to fibers.
The present inventors have found that an internal olefin sulfonate having a particular carbon chain length has the effect of softening fibers, and they have also found that a particular amount or more of a metal ion chelating agent contained in a detergent composition for fibers inhibits the effect of softening fibers by an internal olefin sulfonate having a particular carbon chain length. The present inventors have further found that a detergent composition for fibers containing an internal olefin sulfonate having a particular carbon chain length can finish fibers softly while maintaining the detergency against stains attached to fibers, by limiting the amount of components that have heretofore generally been used for improvement of the detergency.
The present invention relates to a detergent composition for fibers containing the following component (A), component (B) and component (C):

component (A): an internal olefin sulfonate having 17 or more and 24 or less carbon atoms,
component (B): a nonionic surfactant, and
component (C): a metal ion chelating agent,

wherein the mass ratio (B)/(A) of component (B) to component (A) is 0 or more and 1.0 or less, component (B) is a nonionic surfactant having an HLB of more than 10.5 when the composition contains component (B), the content of component (C) is 20% by mass or less, and the composition is a composition for use in washing fibers in water containing a hardness component.

Accordingly, the present invention relates to a detergent composition for fibers containing the following component (A), component (B) and component (C):

component (A): an internal olefin sulfonate having 17 or more and 24 or less carbon atoms,
component (B): a nonionic surfactant, and
component (C): a metal ion chelating agent,

wherein the mass ratio (B)/(A) of component (B) to component (A) is 0 or more and 1.0 or less, the composition contains a nonionic surfactant having an HLB of more than 10.5 [hereinafter referred to as component (B1)] when the composition contains component (B), the content of component (C) is 20% by mass or less, and the composition is a composition for use in washing fibers in water containing a hardness component.

The present invention also relates to a method for washing fibers with a detergent liquid containing the following component (A) and water, wherein the hardness of the detergent liquid is more than 0°dH:
component (A): an internal olefin sulfonate having 17 or more and 24 or less carbon atoms.

The present invention also relates to a method for washing fibers with a detergent liquid containing the following component (A) and water, wherein the hardness of the water in the detergent liquid is more than 0°dH:
component (A): an internal olefin sulfonate having 17 or more and 24 or less carbon atoms.

According to the present invention, it is possible to provide a detergent composition for fibers and a method for washing fibers, which can finish fibers softly while maintaining detergency against stains attached to fibers.

Embodiments of the Invention

<Component (A)>

Component (A) of the present invention is an internal olefin sulfonate having 17 or more and 24 or less carbon atoms and has the effect of improving the softness of fibers. The number of carbon atoms of the internal olefin sulfonate having 17 or more and 24 or less carbon atoms refers to the number of carbon atoms of the internal olefin to which the sulfonate is covalently bonded. The number of carbon atoms of the internal olefin sulfonate having 17 or more and 24 or less carbon atoms is 17 or more and preferably 18 or more, and 24 or less, preferably 22 or less and more preferably 20 or less, from the viewpoint of softening fibers.

The internal olefin sulfonate of the present invention is a sulfonate obtained by sulfonating, neutralizing and hydrolyzing an internal olefin (an olefin having a double bond inside an olefin chain) having 17 or more and 24 or less carbon atoms as a raw material.

Such an internal olefin also includes those containing a trace amount of so-called alpha-olefin (hereinafter also referred to as $\alpha$-olefin) in which the double bond is present at position 1 of the carbon chain. When an internal olefin is sulfonated, $\beta$-sultone is produced quantitatively, and a part of $\beta$-sultone is changed to $\gamma$-sultone and an olefin sulfonic acid, and further converted to a hydroxyalkane sulfonate and an olefin sulfonate in the process of neutralization and hydrolysis (e.g., J. Am. Oil Chem. Soc. 69, 39 (1992)). The hydroxy group of the resulting hydroxyalkane sulfonate is inside the alkane chain, and the double bond of the olefin sulfonate is inside the olefin chain. The resulting product contains mainly a mixture of these, and may contain, in some cases, a trace amount of a hydroxyalkane sulfonate having a hydroxy group at the end of its carbon chain or an olefin sulfonate having a double bond at the end of its carbon chain.

In the present specification, each of these products and a mixture thereof are collectively referred to as "internal olefin sulfonate (component (A)). In addition, "hydroxyalkane sulfonate" is referred to as "hydroxy form of internal olefin sulfonate" (hereinafter also referred to as "HAS"), and "olefin sulfonate" as "olefin form of internal olefin sulfonate" (hereinafter also referred to "IOS").

The mass ratio of HAS to IOS of the compound in component (A) can be measured by high performance liquid chromatography mass spectrometer (hereinafter abbreviated as HPLC-MS). Specifically, the mass ratio can be determined from the HPLC-MS peak area of component (A).

Examples of the salt of the internal olefin sulfonate include an alkali metal salt, an alkaline earth metal (1/2 atom) salt, an ammonium salt or an organic ammonium salt. Examples of the alkali metal salt include a sodium salt and a potassium salt. Examples of the organic ammonium salt include an alkanolammonium salt having 2 or more and 6 or less carbon atoms. From the viewpoint of versatility, the salt of internal olefin sulfonate is preferably an alkali metal salt, and more preferably a sodium salt and a potassium salt.

As is clear from the above-mentioned production method, the sulfonic acid group of the internal olefin sulfonate of component (A) is present inside the carbon chain, that is, the olefin chain or the alkane chain of the internal olefin sulfonate, and a trace amount of the internal olefin sulfonate having a sulfonic acid group at the end of its carbon chain may be, in some cases, contained. In the present invention, the content of the internal olefin sulfonate with the sulfonate group at position 2 in component (A) is preferably 10% by mass or more, more preferably 15% by mass or more, further preferably 20% by mass or more, furthermore preferably 25% by mass or more, furthermore preferably 30% by mass or more, furthermore preferably 35% by mass or more and furthermore preferably 40% by mass or more, and preferably 60% by mass or less in component (A), from the viewpoint of improving the softness of fibers.

The content of the internal olefin sulfonate with the sulfonate group at position 6 or higher in component (A) is preferably 50% by mass or less, more preferably 45% by mass or less, further preferably 40% by mass or less, furthermore preferably 35% by mass or less, furthermore preferably 30% by mass or less, furthermore preferably 25% by mass or less, furthermore preferably 20% by mass or less, furthermore preferably 15% by mass or less, and furthermore preferably 10% or less, from the viewpoint of improving the softness of fibers.

The content of the internal olefin sulfonate with the sulfonate group at position 5 or higher in component (A) is, from the viewpoint of improving the softness of fibers, preferably 60% by mass or less, more preferably 55% by mass or less, further preferably 45% by mass or less, furthermore preferably 40% by mass or less, furthermore preferably 35% by mass or less, furthermore preferably 30% by mass or less, furthermore preferably 25% by mass or less and furthermore preferably 20% by mass or less, and from the viewpoint of ease of production, preferably more than 0% by mass and more preferably 5% by mass or more.

The mass ratio of the content of the internal olefin sulfonate with a sulfonate acid group at position 2 or higher and position 4 or lower to the content of the internal olefin sulfonate with the sulfonate group at position 5 or higher and position 9 or lower in component (A), that is, (content of the internal olefin sulfonate with the sulfonate group at position 2 or higher and position 4 or lower)/(content of the internal olefin sulfonate with the sulfonate group at position 5 or higher and position 9 or lower) is, from the viewpoint of improving the softness of fibers, preferably 0.5 or more, more preferably 0.8 or more, further preferably 1.0 or more, furthermore preferably 1.5 or more, furthermore preferably 2 or more, furthermore preferably 2.5 or more, furthermore preferably 3 or more, furthermore preferably 4 or more and furthermore preferably 4.5 or more, and from the viewpoint of ease of production, preferably 20 or less, more preferably 10 or less, further preferably 8 or less and furthermore preferably 6 or less.

The content of each of compounds with the sulfonate group at different positions in component (A) can be measured by HPLC-MS. In the present specification, the content of each of compounds with the sulfonate group at different positions will be determined as the mass ratio of the compound with the sulfonate group at each position in all HAS forms of component (A), based on the HPLC-MS peak area.

The content of the olefin sulfonate with the sulfonate group at position 1 in component (A) is, from the viewpoint of imparting good softness to fibers even when the temperature of the water used for washing is a low temperature of 0°C or more and 15°C or less, preferably 10% by mass or less, more preferably 7% by mass or less, further preferably 5% by mass or less and furthermore preferably 3% by mass or less, and from the viewpoint of reducing production cost and improving productivity, preferably 0.01% by mass or more in component (A).

The position of the sulfonate group in these compounds is the position in the olefin chain or the alkane chain.

The internal olefin sulfonate can be a mixture of the hydroxy form and the olefin form. The mass ratio (olefin form/hydroxy form) of the content of the olefin form of internal olefin sulfonate to the content of the hydroxy form of internal olefin sulfonate in component (A) can be 0/100 or more and further 5/95 or more, and 50 / 50 or less, further 40/60 or less, further 30/70 or less and further 25/75 or less.

The mass ratio of the content of the hydroxy form of internal olefin sulfonate and the content of the olefin form of internal olefin sulfonate in component (A) can be measured by the method described in Examples after separating component (A) into the hydroxy form and the olefin form by high performance liquid chromatography (HPLC).

Component (A) can be produced by sulfonating, neutralizing and hydrolyzing an internal olefin having 17 or more and 24 or less carbon atoms as a raw material. The sulfonation reaction can be carried out by allowing 1.0 to 1.2 mol of sulfur trioxide gas to react with 1 mol of the internal olefin. The reaction can be carried out at a reaction temperature of 20 to 40°C.

The neutralization is carried out by allowing an aqueous solution of alkali such as sodium hydroxide, ammonia or 2-aminoethanol to react with the sulfonate group in an amount of 1.0 to 1.5 molar times the theoretical value of the sulfonate group. The hydrolysis reaction may be carried out at 90 to 200°C for 30 minutes to 3 hours in the presence of water. These reactions can be carried out continuously. After completion of the reaction, purification can be carried out by extraction, washing or the like.

In producing internal olefin sulfonate (A), sulfonation, neutralization and hydrolysis processes may be carried out using an internal olefin having a distribution of 17 or more and 24 or less carbon atoms as a raw material; sulfonation, neutralization and hydrolysis processes may be carried out using an internal olefin having a single number of carbon atoms as a raw material; or if necessary, plural types of internal olefin sulfonate having different numbers of carbon atoms which have previously produced may be mixed.

In the present invention, the internal olefin refers to an olefin having a double bond inside the olefin chain as described above. The number of carbon atoms of the internal olefin of component (A) is 17 or more and 24 or less. The internal olefin used in component (A) may be used alone or in combination of two or more.

When an internal olefin as a raw material is sulfonated, neutralized and hydrolyzed to obtain an internal olefin sulfonate of component (A) containing a particular amount of component (a1), the total content of the internal olefin having a double bond at position 2 in the internal olefin as a raw material is preferably 10% by mass or more, more preferably 15% by mass or more, further preferably 20% by mass or more, furthermore preferably 25% by mass or more, furthermore preferably 30% by mass or more, furthermore preferably 35% by mass or more and furthermore preferably 40% by mass or more, and preferably 60% by mass or less, from the viewpoint of improving the softness of fibers.

The total content of an olefin having a double bond at position 1, so-called alpha-olefin in internal olefin as a raw material is, from the viewpoint of imparting good softness to fibers even when the temperature of the water used for washing is a low temperature of 0°C or more and 15°C or less, preferably 10% by mass or less, more preferably 7% by mass or less, further preferably 5% by mass or less and furthermore preferably 3% by mass or less, and from the viewpoint of reducing production cost and improving productivity, preferably 0.01% by mass or more.

The content of the olefin having a double bond at position 6 or higher in the internal olefin as a raw material is preferably 50% by mass or less, more preferably 45% by mass or less, further preferably 40% by mass or less, furthermore preferably 35% by mass or less, furthermore preferably 30% by mass or less, furthermore preferably 25% by mass or less, furthermore preferably 20% by mass or less, furthermore preferably 15% by mass or less and furthermore preferably 10% by mass or less, from the viewpoint of improving the softness of fibers. The highest position at which the double bond occurs in the internal olefin as a raw material varies depending on the number of carbon atoms.

Distribution of a double bond in the olefin as a raw material can be measured, for example, by gas chromatograph mass spectrometer (hereinafter abbreviated as GC-MS). Specifically, each component different in the carbon chain length and the double bond position is precisely separated from each other by a gas chromatograph analyzer (hereinafter abbreviated as GC), and each component can be subjected to a mass spectrometer (hereinafter abbreviated as MS) to identify the double bond position, and the percentage of each component can be determined from its GC peak area.

<Component (B)>

Component (B) is a nonionic surfactant. Component (B) is an optional component, and when the detergent composition for fibers of the present invention contains component (B), it contains a nonionic surfactant [component (B1)] having an HLB of more than 10.5.

A preferred component (B1) is a nonionic surfactant containing a polyoxyethylene group and having an HLB of more than 10.5 and 19 or less. From the viewpoint of improving the effect of softening fibers of component (A), the HLB of component (B1) blended in the present invention is preferably 11 or more, more preferably 12 or more, further preferably 13 or more, furthermore preferably 14 or more, furthermore preferably 15 or more and furthermore preferably 16 or more, and preferably 19 or less.

The value of the HLB of component (B1) in present invention refers to the HLB calculated by the following formula when component (B1) contains polyoxyethylene group. The average molecular weight of the polyoxyethylene group refers to the average molecular weight calculated from the average number of added moles when the molar number of added oxyethylene group has a distribution. The average molecular weight of component (B1) refers to the molecular weight calculated as an average value when a hydrophobic group such as a hydrocarbon group has a distribution or the mole number of the polyoxyethylene group added has a distribution.

$$\texttt{HLB = [(average molecular weight of polyoxyethylene}$$

$$\texttt{group)/(average molecular weight of component (B1)] × 20}$$

Hereinafter, specific nonionic surfactants will be illustrated, but the above-mentioned "oxyethylene group" may be sometimes referred to as "ethyleneoxy group".

In the present invention, when the nonionic surfactant contains no polyoxyethylene group, the HLB of the nonionic surfactant refers to a value measured according to the method of Kunieda et al. described in "Journal of Colloid and Interface Science, Vol. 107, No. 1, September 1985". This document describes a measurement method of an HLB based on the finding that there is a linear relationship between a particular temperature ($T_{HLB}$) and the number of HLB by Griffin.

Component (B1) is suitably a nonionic surfactant having an HLB of more than 10.5 and preferably 19 or less and is represented by the following general formula (b1):

$$R^1(CO)_mO\text{-}(A^1O)_n\text{-}R^2 \qquad (b1)$$

wherein $R^1$ is an aliphatic hydrocarbon group having 9 or more and 16 or less carbon atoms, $R^2$ is a hydrogen atom or a methyl group, CO is a carbonyl group, m is a number of 0 or 1, $A^1O$ group is one or more groups selected from an ethyleneoxy group and a propyleneoxy group, and n is an average number of added moles and is 6 or more and 50 or less.

In the general formula (b1), $R^1$ is an aliphatic hydrocarbon group having 9 or more and 16 or less carbon atoms. The value of the HLB is lower as the number of carbon atoms of $R^1$ is more and is higher as the number of carbon atoms of $R^1$ is less. The carbon number of $R^1$ is, from the viewpoint of allowing stains attached to fibers to be easily removed, 9 or more, preferably 10 or more and more preferably 11 or more, and from the viewpoint of further increasing the effect of softening fibers, 16 or less, preferably 15 or less and more preferably 14 or less.

The aliphatic hydrocarbon group of $R^1$ is preferably a group selected from an alkyl group and an alkenyl group.

In the general formula (b1), the $A^1O$ group is one or more groups selected from an ethyleneoxy group and a propyleneoxy group. When an ethyleneoxy group and a propyleneoxy group are contained, the ethyleneoxy group and the propyleneoxy group may be bonded in block type or random type. From the viewpoint of avoiding the effect of softening fibers of component (A) from being inhibited, the $A^1O$ group is preferably a group containing an ethyleneoxy group. When the $A^1O$ group is an ethyleneoxy group, the HLB value is higher than that when it is a propyleneoxy group.

In the general formula (b1), n is an average number of added moles, and is 6 or more and 50 or less. The value of the HLB is higher as the number of n is more and is lower as the number of n is less. From the viewpoint of avoiding the effect of softening fibers of component (A) from being inhibited, n is 6 or more, preferably 6.5 or more, more preferably 7 or more, further preferably 8 or more, furthermore preferably 9 or more, furthermore preferably 10 or more and furthermore preferably 12 or more, and from the viewpoint of the detergent property against stains attached to fibers, n is 50 or less, preferably 45 or less, more preferably 40 or less, further preferably 35 or less, furthermore preferably 26 or less and furthermore preferably 24 or less.

When the detergent composition for fibers of the present invention contains component (B), it always also contains component (B1). When the detergent composition for fibers of the present invention contains component (B), the content of component (B1) in component (B) is preferably 60% by mass or more, more preferably 70% by mass or more, further preferably 80% by mass or more and furthermore preferably 90% by mass or more, and preferably 100% by mass or less, and it may be 100% by mass.

The detergent composition for fibers of the present invention may contain a nonionic surfactant other than component (B1), i.e., a nonionic surfactant having an HLB of 10.5 or less, as component (B). Examples of the nonionic surfactant having an HLB of 10.5 or less include a nonionic surfactant, represented by the above general formula (b1), the structure of which is changed so that the HLB is 10.5 or less.

<Component (C)>

Component (C) is a metal ion chelating agent. The detergent composition for fibers of the present invention optionally contains a metal ion chelating agent which is component (C). Inclusion of a metal ion chelating agent in a detergent composition for fibers is commonly carried out in order to improve the detergent property. However, the present inventors have found that fibers can be finished softly by limiting the content of the metal ion chelating agent in a detergent composition for fibers. Component (C) of the present invention is preferably a metal ion chelating agent capable of chelating a divalent or higher metal ion. Component (C) of the present invention is preferably one or more metal ion chelating agents selected from (C1) a metal ion chelating agent which is an inorganic compound and (C2) metal ion chelating agent which is an organic compound.

[(C1) Metal ion chelating agent which is an inorganic compound]

Component (C1) is preferably one or more selected from (C1-1) an alkali metal silicate, (C1-2) an aluminosilicate and (C1-3) a tripolyphosphate.

[(C1-1) Alkali metal silicate]

The alkali metal silicate is an alkali metal salt of silicic acid ($SiO_2$), and a compound of an alkali metal silicate in which a $SiO_2/M_2O$ wherein M represents an alkali metal is 0.5 to 2.6 is generally used. More specifically, it has a composition represented by the following general formula (c1-1):

$$x(M_2O) \cdot y(SiO_2) \cdot z(Me_mO_n) \cdot w(H_2O) \qquad \text{(c1-1)}$$

wherein M represents one or a combination of two or more selected from the group consisting of alkali metals, Me is one or two or more elements selected from the elements of the Groups II, III, IV and VIII of the periodic table of elements, y/x = 0.5 or more and 2.6 or less, z/x = 0.01 or more and 10 or less, w = 0 or more and 20 or less, and n/m = 0.5 or more and 20 or less.

Examples of the alkali metal M in the general formula (c1-1) include Na and K. These may be used alone or, for example, $Na_2O$ and $K_2O$ may be mixed to constitute the $M_2O$ component. Examples of Me include Mg, Ca, Zn, Y, Ti, Zr and Fe. These are not particularly limited, but from the viewpoint of resources and safety, are preferably Mg and Ca. These may be used alone or in mixture of two or more thereof, and, for example, MgO, CaO or the like may be mixed to constitute a $Me_mO_n$ component.

In the general formula (c1-1), y/x is 0.5 or more and preferably 1.5 or more, and 2.6 or less, and 2.2 or less. When y/x exceeds 2.6, the ion exchange capacity becomes low. Also, in the general formula (c1-1), when z/x exceeds 1.0, the ion exchange capacity becomes low. Further, x, y and z are not particularly limited as long as they have such a relationship as given by y/x ratio and z/x ratio described above. When x ($M_2O$) is, for example, x'($Na_2O$)·X"($K_2O$) as described above, x is x' + x". Such a relationship also applies to z in the case of a z($Me_mO_n$) component composed of two or more components. n/m represents the number of oxygen ions coordinated to the element(s), and is substantially selected from values of 0.5, 1.0, 1.5 and 2.0.

The alkali metal silicate represented by the general formula (c1-1) preferably has an ion exchange capacity of 100 $CaCO_3$mg/g or more and more preferably 200 to 600 $CaCO_3$mg/g. The alkali metal silicate represented by the general formula (c1-1) is one of the substances having an ion capture capacity in the present invention.

[(C1-2) Aluminosilicate]

The aluminosilicate may be either crystalline or amorphous, but is preferably a crystalline aluminosilicate, as a divalent compound having a high metal ion-exchange capture capacity. The crystalline aluminosilicate is generally referred to as zeolite, and more specifically, it has a composition represented by the following general formula (c1-2-1):

$$a'(M_2O)·Al_2O_3·b'(SiO_2)·w(H_2O) \qquad (c1-2-1)$$

wherein M represents an alkali metal atom, a', b' and w each represent the molar number of each component, and generally, a' is 0.7 or more and 1.5 or less, b' is 0.8 or less and less than 6, and w is an arbitrary positive number.

Among crystalline aluminosilicates having the general formula (c1-2-1), those represented by the following general formula (c1-2-2) are preferred:

$$Na_2O·Al_2O_3·n(SiO_2)·w(H_2O) \qquad (c1-2-2)$$

wherein n represents a number of 1.8 or more and 3.0 or less, and w represents a number of 1 or more and 6 or less.

As a crystalline aluminosilicate (zeolite), a synthetic zeolite having an average primary particle diameter of 0.1 $\mu$m or more and 10 $\mu$m or less, typically type A-, type X- and type P-zeolite, is preferably used. The zeolite may be blended as agglomerated dried zeolite particles obtained by drying a zeolite powder and/or a zeolite slurry.

[(C1-3) Tripolyphosphate]

One specific example of the metal ion chelating agent which is an inorganic compound is one or more tripolyphosphates. Examples of the counter ion that forms the salt of tripolyphosphate include an ion selected from a sodium ion and a potassium ion.

[(C2) Metal ion chelating agent which is an organic compound]

Examples of the metal ion chelating agent which is an organic compound usable in the present invention include one or more organic compounds selected from:

(C2-1) a divalent or higher and tetravalent or lower carboxylic acid having 4 or more and 12 or less carbon atoms and containing no amino group, or a salt thereof,
(C2-2) a divalent or higher and tetravalent or lower carboxylic acid having 4 or more and 10 or less carbon atoms and containing an amino group, or a salt thereof, and
(C2-3) a compound having a phosphonic acid group or a salt thereof in the molecule.

Specific examples of component (C2-1) include one or more carboxylic acids selected from citric acid, tartaric acid, succinic acid and malic acid or salts thereof.

Specific examples of component (C2-2) include one or more carboxylic acids selected from nitrilotriacetic acid, ethylenediaminetetraacetic acid, 3-hydroxy-2,2'-iminodisuccinic acid, diethylenetriaminepentaacetic acid and hydroxymethylethylenediaminetriacetic acid or salts thereof.

Examples of component (C2-3) include organic phosphonic acid derivatives such as ethane-1,1-diphosphonic acid, ethane-1,1,2-triphosphonic acid, 1-hydroxyethylidene-1,1-diphosphonic acid, ethanehydroxy-1,1,2-triphosphonic acid, ethane-1,2-dicarboxy-1,2-diphosphonic acid, methane hydroxyphosphonic acid, nitrilotrimethylene phosphonic acid and ethylenediamine tetrakis(methylenephosphonic acid).

From the viewpoint of versatility, the salts of component (C2-1) to component (C2-3) are preferably salts of an alkali metal such as Na or K and an alkanolamine having 2 or more and 6 or less carbon atoms such as monoethanolamine, diethanolamine or triethanolamine.

<Composition and others>

The content of component (A) in the detergent composition for fibers of the present invention is, from the viewpoint of further improving the effect of imparting softness to fibers per mass of the detergent composition for fibers during washing of fibers, preferably 4% by mass or more, more preferably 5% by mass or more, further preferably 6% by mass or more, furthermore preferably 7% by mass or more, furthermore preferably 8% by mass or more and furthermore preferably 9% by mass or more; and from the viewpoint of blending cost, preferably 70% by mass or less, more preferably 60% by mass or less and further preferably 50% by mass or less.

The content of component (A) contained in the detergent composition for fibers is based on the value calculated assuming that the counter ion is a sodium ion. That is the content calculated based on the form of a sodium salt.

In the detergent composition for fibers of the present invention, the mass ratio (B)/(A) of the content of component (B) to the content of component (A) is, from the viewpoint of further improving the detergent property against stains attached to fibers per mass of the detergent composition for fibers, 0 or more, preferably 0.05 or more, more preferably 0.08 or more, further preferably 0.1 or more, furthermore preferably 0.15 or more and furthermore preferably 0.2 or more, and from the viewpoint of further improving the effect of imparting softness to fibers per mass of the detergent composition for fibers during washing of fibers, 1.0 or less, preferably 0.9 or less, more preferably 0.8 or less, further preferably 0.7 or less, furthermore preferably 0.6 or less, furthermore preferably 0.5 or less, furthermore preferably 0.45 or less, furthermore preferably 0.40 or less, furthermore preferably 0.35 or less and furthermore preferably 0.30 or less.

The content of component (C) in the detergent composition for fibers of the present invention is, from the viewpoint of further improving the effect of imparting softness to fibers per mass of the detergent composition for fibers during washing of fibers, 20% by mass or less, preferably 18% by mass or less, more preferably 15% by mass or less, further preferably 10% by mass or less, furthermore preferably 7% by mass or less, furthermore preferably 5% by mass or less, furthermore preferably 4% by mass or less, furthermore preferably 3% by mass or less, furthermore preferably 1% by mass or less and furthermore preferably 0.5% by mass or less, and from the viewpoint of further improving the detergent property against stains attached to fibers, preferably more than 0% by mass and more preferably 0.5% by mass or more. It is preferable that the content of component (C) in the detergent composition for fibers of the present invention is 0% by mass, i.e., the detergent composition has no component (C).

The content of component (C) contained in the detergent composition for fibers is based on the value calculated assuming that the counter ion is H, i.e., an acid type.

In the detergent composition for fibers of the present invention, the mass ratio (C)/(A) of the content of component (C) to the content of component (A) is, from the viewpoint of further improving the effect of imparting softness to fibers per mass of the detergent composition for fibers, preferably 2 or less, more preferably 1.5 or less, further preferably 1 or less, furthermore preferably 0.8 or less, furthermore preferably 0.7 or less, furthermore preferably 0.6 or less and furthermore preferably 0.5 or less, and 0 or more, and it may be 0.

The detergent composition for fibers of the present invention is suitable for washing fibers in water containing a hardness component. The above-mentioned "suitable for washing fibers in water containing a hardness component" means that by washing fibers in water containing a hardness component with the detergent composition for fibers of the present invention, fibers can be finished softly and stains attached to the fibers can be washed off.

From the viewpoint of further improving the effect of imparting softness to fibers, the hardness of water containing a hardness component used for washing is, by German hardness, preferably 1°dH or more, more preferably 2°dH or more and further preferably 3°dH or more, and preferably 20°dH or less, more preferably 18°dH or less and further preferably 15°dH or less. The German hardness (°dH) used in the present specification refers to the concentrations of calcium and magnesium in water expressed as the concentration calculated based on the form of $CaCO_3$: 1 mg/L (ppm) = about 0.056°dH (1°dH = 17.8 ppm) .

The concentrations of calcium and magnesium for this German hardness are determined by a chelate titration method using disodium ethylenediaminetetraacetate salt.

A specific method for measuring the German hardness of water in the present specification is shown as follows.

<Method for measuring German hardness of water>

[Reagent]

- 0.01 mol/l EDTA·2Na solution: a 0.01 mol/l aqueous solution of disodium ethylenediaminetetraacetate (a titration solution, 0.01 M EDTA-Na2, manufactured by SIGMA-ALDRICH)
- Universal BT indicator (product name: Universal BT, manufactured by Dojindo Laboratories)
- Ammonia buffer solution for hardness measurement (a solution prepared by dissolving 67.5 g of ammonium chloride in 570 ml of 28 w/v% ammonia water and adding ion-exchanged water until the total volume is 1000 ml)

[Measurement of hardness]

(1) 20 ml of water serving as a sample is collected in a conical beaker with a whole pipette.
(2) 2 ml of an ammonia buffer solution for hardness measurement is added thereto.
(3) 0.5 ml of Universal BT indicator is added thereto. It is made sure that the solution after addition is reddish violet.
(4) While shaking the conical beaker well, a 0.01 mol/l EDTA·2Na solution is added dropwise thereto from a burette, and the point at which the sample water turns blue is taken as the end point of the titration.
(5) The total hardness is determined by the following calculation formula:

$$\text{Hardness (}^{\circ}\text{dH)} = T \times 0.01 \times F \times 56.0774 \times 100/A$$

wherein:

T: Titer of a 0.01 mol/l EDTA·2Na solution (mL),

A: Sample volume (20 mL, a volume of sample water), and

F: Factor of a 0.01 mol/l EDTA·2Na solution.

<Fibers>

The fiber to be washed with the detergent composition for fibers of the present invention may be either a hydrophobic fiber or a hydrophilic fiber. Examples of the hydrophobic fiber include a protein-based fiber (such as cow milk protein casein fiber or promix), a polyamide-based fiber (such as nylon), a polyester-based fiber (such as polyester), a polyacrylonitrile-based fiber (such as acrylic), a polyvinyl alcohol-based fiber (such as vinylon), a polyvinyl chloride-based fiber (such as polyvinyl chloride), a polyvinylidene chloride-based fiber (such as vinylidene), a polyolefin-based fiber (such as polyethylene or polypropylene), a polyurethane-based fiber (such as polyurethane), a polyvinyl chloride/polyvinyl alcohol copolymer-based fiber (such as polychlal), a polyalkylene paraoxybenzoate-based fiber (such as benzoate), a polyfluoroethylene-based fiber (such as polytetrafluoroethylene), a glass fiber, a carbon fiber, an alumina fiber, a silicon carbide fiber, a rock fiber, a slag fiber and a metal fiber (a gold thread, a silver thread or a steel fiber). Examples of the hydrophilic fiber include a seed hair fiber (such as cotton, arboreous cotton or kapok), a bast fiber (such as linen, flax, ramie, hemp or jute), vein fiber (such as manila hemp or sisal hemp), coconut fiber, rush, straw, an animal hair fiber(such as wool, mohair, cashmere, camel hair, alpaca, vicuna or angora), a silk fiber (domesticated silkworm silk or wild silkworm silk), a feather and down and a cellulosic fiber (such as rayon, polynosic, cupra or acetate).

From the viewpoint that the softness of fibers after washing with the detergent composition for fibers of the present invention is more easily felt, the fiber is preferably a fiber containing a cotton fiber. From the viewpoint of further improving the softness of fibers, the content of the cotton fiber in the fiber is preferably 5% by mass or more, more preferably 10% by mass or more, further preferably 15% by mass or more, furthermore preferably 20% by mass or more and furthermore preferably 100% by mass or more.

<Textile product>

In the present invention, the textile product refers to a cloth produced by using the above-mentioned hydrophobic fiber or hydrophilic fiber such as a woven fabric, a knitted fabric or a nonwoven fabric, and a product obtained by using the cloth such as an undershirt, a T-shirt, a business shirt, a blouse, pants, a hat, a handkerchief, a towel, a knit, socks, an underwear or tights. From the viewpoint that the softness of fibers after washing with the detergent composition for fibers of the present invention is more easily felt, the textile product is preferably a textile product containing a cotton fiber. From the viewpoint of further improving the softness of fibers, the content of the cotton fiber in the textile product is preferably 5% by mass or more, more preferably 10% by mass or more, further preferably 15% by mass or more, furthermore preferably 20% by mass or more and furthermore preferably 100% by mass or more.

<Optional components>

[Component (D): surfactant other than component (A)]

Surfactants other than component (A) can be used as component (D) in the detergent composition for fibers of the present invention, as long as they do not interfere with the effect of the present invention. Examples of component (D) include one or more anionic surfactants selected from the following component (d1), component (d2), component (d3) and component (d4):

component (d1): alkyl or alkenyl sulfate,
component (d2): polyoxyalkylene alkyl ether sulfate or polyoxyalkylene alkenyl ether sulfate,
component (d3): an anionic surfactant having a sulfonate group (except for component (A)), and
component (d4): a fatty acid or a salt thereof.

Examples of component (D) other than those described above include component (d5) which is a nonionic surfactant having a hydroxy group or polyoxyalkylene group.

Specific examples of component (d1) include one or more anionic surfactants selected from alkyl sulfates having an alkyl group having 10 or more and 18 or less carbon atoms and alkenyl sulfates having an alkenyl group having 10 or more and 18 or less carbon atoms. From the viewpoint of improving the detergent property, component (d1) is preferably one or more anionic surfactants selected from alkyl sulfates having an alkyl group having 12 or more and 14 or less carbon atoms, and more preferably one or more anionic surfactants selected from sodium alkyl sulfates having an alkyl group having 12 or more and 14 or less carbon atoms.

Specific examples of component (d2) include one or more anionic surfactants selected from a polyoxyalkylene alkyl sulfate having an alkyl group having 10 or more and 18 or less carbon atoms and having an average number of moles of added alkylene oxide of 1 or more and 3 or less, and a polyoxyalkylene alkenyl ether sulfate having an alkenyl group having 10 or more and 18 or less carbon atoms and having an average number of moles of added alkylene oxide of 1 or more and 3 or less. From the viewpoint of improving the detergent property, component (d2) is preferably a polyoxyethylene alkyl sulfate having an average number of moles of added ethylene oxide of 1 or more and 2.2 or less, more preferably a polyoxyethylene alkyl sulfate having an alkyl group having 12 or more and 14 or less carbon atoms and having an average number of moles of added ethylene oxide of 1 or more and 2.2 or less, and further preferably sodium salts thereof.

An anionic surfactant having a sulfonate group as component (d3) refers to an anionic surfactant having a sulfonate as a hydrophilic group (except for component (A)).

Specific examples of component (d3) include one or more anionic surfactants selected from an alkylbenzene sulfonate having an alkyl group having 10 or more and 18 or less carbon atoms, an alkenylbenzene sulfonate having an alkenyl group having 10 or more and 18 or less carbon atoms, an alkane sulfonate having an alkyl group having 10 or more and 18 or less carbon atoms, an $\alpha$-olefin sulfonate having an $\alpha$-olefin moiety having 10 or more and 18 or less carbon atoms, an $\alpha$-sulfofatty acid salt having a fatty acid moiety having 10 or more and 18 or less carbon atoms, an $\alpha$-sulfofatty acid lower alkyl ester salt having a fatty acid moiety having 10 or more and 18 or less carbon atoms and an ester moiety having 1 or more and 5 or less carbon atoms, and an internal olefin sulfonate having 12 or more and 16 or less carbon atoms. From the viewpoint of improving the detergent property, component (d3) is preferably an alkylbenzene sulfonate having an alkyl group having 11 or more and 14 or less carbon atoms, and more preferably a sodium alkylbenzene sulfonate having an alkyl group having 11 or more and 14 or less carbon atoms.

Examples of a fatty acid or a salt thereof as component (d4) include a fatty acid or a salt thereof having 10 or more and 20 or less carbon atoms. From the viewpoint of further increasing the effect of softening fibers of component (A), the number of carbon atoms of component (d4) is 10 or more, preferably 12 or more and more preferably 14 or more, and 20 or less and preferably 18 or less.

The salt of an anionic surfactant as components (d1) to (d4) is preferably an alkali metal salt, more preferably a sodium salt or a potassium salt, and further preferably a sodium salt.

When the detergent composition for fibers of the present invention contains one or more anionic surfactants selected from component (d1), component (d2), component (d3) and component (d4), the content of component (A) in the total mass of component (A) and components (d1) to (d4) is, from the viewpoint of improving the softness of fibers, preferably 50% by mass or more, more preferably 60% by mass or more, furthermore preferably 70% by mass or more, furthermore preferably 80% by mass or more and furthermore preferably 90% by mass or more, and 100% by mass or less.

The mass of component (A) or the anionic surfactant other than component (A) is determined by using the value of the counter ion of the anionic surfactant calculated based on the form of a sodium ion.

[Component (E): Alkali agent]

The detergent composition for fibers of the present invention can contain an alkali agent as component (E) from the viewpoint of further improving the detergent property against stains attached to fibers. Specific examples of the alkali agent can include one or more inorganic alkali agents selected from sodium carbonate, potassium carbonate, sodium sesquicarbonate and sodium hydrogen carbonate. The inorganic alkali agent is preferably one or more alkali agents selected from sodium carbonate and potassium carbonate, and more preferably sodium carbonate. Examples of the alkali agent other than those described above can include an alkanolamine in which among the groups attached to a nitrogen bond, one or more and three or less groups are alkanol groups having 2 or more and 4 or less carbon atoms and the other(s) are an alkyl group having 1 or more and 4 or less carbon atoms or a hydrogen atom. Among them, the alkanol group of the alkanolamine is preferably a hydroxyalkyl group and further preferably a hydroxyethyl group. Except for the alkanol group, a hydrogen atom or methyl group is preferred, and a hydrogen atom is particularly preferred. Examples of the alkanolamine include an alkanolamine such as 2-aminoethanol, N-methylethanolamine, N,N-dimethylethanolamine, N,N-diethylethanolamine, diethanolamine, N-methyldiethanolamine and triethanolamine. In the present invention, component (E) is preferably an alkanolamine selected from monoethanolamine and triethanolamine and more preferably monoethanolamine.

<Component (F)>

The detergent composition for fibers of the present invention can further contain an organic solvent having one or more hydroxy groups as component (F). Examples of the organic solvent having one or more hydroxy groups include one or more organic solvents selected from monohydric or higher and hexahydric or lower alcohols having an aliphatic hydrocarbon group having 2 or more and 6 or less carbon atoms such as ethanol, 1-propanol, 2-propanol, ethylene glycol, propylene glycol, butylene glycol, 2-methyl-2,4-pentanediol, 1,5-pentanediol, 1,6-hexanediol, glycerin or 2-methyl-2,4-pentanediol.

<Water>

In order to bring the detergent composition for fibers of the present invention into a liquid state at 4°C or more and 40°C or less, water can be contained therein. Water to be used can be deionized water (sometimes also referred to as ion-exchanged water) or ion-exchanged water having sodium hypochlorite added at 1 mg/kg or more and 5 mg/kg or less thereto. Tap water can be also used.

In the detergent composition for fibers of the present invention, the content of water is preferably 4% by mass or more and more preferably 5% by mass or more, and preferably 85% by mass or less and more preferably 80% by mass or less.

In addition to these components, the following components (g1) to (g7) may be blended into the detergent composition for fibers of the present invention:

(g1) 0.01% by mass or more and 10% by mass or less of an anti-stain redeposition agent and a dispersing agent such as polyacrylic acid, polymaleic acid or carboxymethyl cellulose,
(g2) 0.01% by mass or more and 10% by mass or less of a bleaching agent such as hydrogen peroxide, sodium percarbonate or sodium perborate,
(g3) 0.01% by mass or more and 10% by mass or less of a bleaching activator such as tetraacetylethylenediamine or bleaching activators represented by the general formulas (1-2) to (1-7) described in JP-A 6-316700,
(g4) 0.001% by mass or more, preferably 0.01% by mass or more, more preferably 0.1% by mass or more and more preferably 0.3% by mass or more, and 2% by mass or less and preferably 1% by mass or less of one or more enzymes selected from cellulase, amylase, pectinase, protease and lipase and preferably one or more enzymes selected from amylase and protease,

(g5) 0.001% by mass or more and 1% by mass or less of a fluorescent dye such as a fluorescent dye commercially available as a Tinopal CBS (trade name, manufactured by Ciba Specialty Chemicals) or Whitex SA (trade name, manufactured by Sumitomo Chemical Co., Ltd.),

(g6) 0.01% by mass or more and 2% by mass or less of an antioxidant such as butylhydroxytoluene, distyrenated cresol, sodium sulfite or sodium hydrogen sulfite, and

(g7) an appropriate amount of a pigment, a perfume, an antimicrobial preservative or a defoaming agent such as silicone.

From the viewpoint of further improving the detergent performance against stains attached to fibers, the pH of the detergent composition for fibers of the present invention at 20°C is preferably 3 or more and more preferably 3.5 or more, and preferably 9 or less and more preferably 8 or less.

<Method for washing fibers>

The method for washing fibers of the present invention is a method for washing fibers with a detergent liquid containing component (A) and water, wherein the hardness of the detergent liquid is more than 0°dH.

In addition, the method for washing fibers of the present invention is a method for washing fibers with a detergent liquid obtained by mixing component (A) and water, wherein the hardness of the water is more than 0°dH.

Further, the method for washing fibers of the present invention is a method for washing fibers with a detergent liquid containing component (A) and water and having a hardness of more than 0°dH.

In these methods for washing fibers of the present invention, the hardness of a detergent liquid is a value calculated by using the "method for measuring the German hardness of water" described above. The hardness of a detergent liquid can also be selected from the preferred range of the hardness of water containing a hardness component described with respect to the detergent composition for fibers of the present invention. The hardness of a detergent liquid can be measured in the same way as the hardness of water. The hardness of water used in the washing method, such as water used for preparing a detergent liquid or water used for rinsing can also be selected from the preferred range of the hardness of water containing a hardness component described with respect to the detergent composition for fibers of the present invention. The hardness of such water can be measured in the same way as the hardness of water described above.

In the method for washing fibers of the present invention, the detergent liquid is preferably one obtained by using the detergent composition for fibers of the present invention.

The matters described with respect to the detergent composition for fibers of the present invention can be appropriately applied to a method for washing fibers of the present invention.

The content of component (A) in the detergent liquid is preferably 0.005% by mass or more and more preferably 0.008% by mass or more, and preferably 1.0% by mass or less and more preferably 0.8% by mass or less.

The detergent liquid optionally contains component (B) and component (C).

In the detergent liquid, the mass ratio (B)/(A) of the content of component (B) to the content of component (A) is, from the viewpoint of further improving the detergent property against stains attached to fibers per mass of the detergent liquid, 0 or more, preferably 0.05 or more, more preferably 0.08 or more, further preferably 0.1 or more, furthermore preferably 0.15 or more and furthermore preferably 0.2 or more, and from the viewpoint of further improving the effect of imparting softness to fibers per mass of the detergent liquid during washing of fibers, 1.0 or less, preferably 0.9 or less, more preferably 0.8 or less, further preferably 0.7 or less, furthermore preferably 0.6 or less, furthermore preferably 0.5 or less, furthermore preferably 0.45 or less, furthermore preferably 0.40 or less, furthermore preferably 0.35 or less and furthermore preferably 0.30 or less.

In the detergent liquid, the mass ratio (C)/(A) of the content of component (C) to the content of component (A) is, from the viewpoint of further improving the effect of imparting softness to fibers per mass of the detergent composition for fibers, preferably 2 or less, more preferably 1.5 or less, further preferably 1 or less, furthermore preferably 0.8 or less, furthermore preferably 0.7 or less, furthermore preferably 0.6 or less and furthermore preferably of 0.5 or less, and 0 or more, and it may be 0.

The temperature of the detergent liquid is, from the viewpoint of further improving the detergent property against stains attached to fibers, preferably 0°C or more, more preferably 3°C or more and further preferably 5°C or more, and from the viewpoint of finishing fibers more softly without excessively removing the oil agent contained in the fibers themselves constituting clothing, preferably 40°C or less and more preferably 35°C or less.

The pH of the detergent liquid at 20°C is, from the viewpoint of further improving the detergent property against stains attached to fibers, preferably 3 or more and more preferably 4 or more, and from the viewpoint of finishing fibers more softly, preferably 10 or less and more preferably 9 or less. The pH can be measured by the following measurement method.

<pH Measurement method>

A pH measuring composite electrode (glass fitting sleeve-type, manufactured by HORIBA, Ltd.) is connected to a pH meter (pH/ion meter F-23, manufactured by HORIBA, Ltd.) and the power is turned on. A saturated potassium chloride aqueous solution (3.33 mol/L) is used as an internal liquid for pH electrode. Next, each of a pH 4.01 standard solution (a phthalate standard solution), a pH 6.86 standard solution (a neutral phosphate standard solution) and a pH 9.18 standard solution (a borate standard solution) is filled in a 100 mL beaker, and immersed in a thermostat bath at 25°C for 30 minutes. The pH measuring electrode is immersed for 3 minutes in each of the standard solutions adjusted to a constant temperature, and subjected to calibration operation in the order of pH 6.86 → pH 9.18 → pH 4.01. Each of samples to be measured is adjusted to 25°C, the electrode of the pH meter is immersed in the sample, and the pH after 1 minute is measured.

Recently, washing machines have become larger and the value of the bath ratio expressed as the ratio of the amount of water (liter) in a detergent liquid to the mass (kg) of clothing, that is, the amount of water (liter) in a detergent liquid/the mass (kg) of clothing (hereinafter also referred to "bath ratio") tends to decrease. When using a household washing machine, the smaller bath ratio leads to the more rubbing between fibers by stirring during washing, sometimes impairing the softness of fibers. According to the method for washing fibers of the present invention, fibers can be finished softly even under washing conditions of a small bath ratio. The bath ratio is, from the viewpoint of finishing fibers more softly, preferably 2 or more, more preferably 3 or more, further preferably 4 or more and furthermore preferably 5 or more, and from the viewpoint of maintaining detergency, preferably 45 or less, more preferably 40 or less, further preferably 30 or less and furthermore preferably 20 or less.

According to the method for washing fibers of the present invention, fibers can be finished more softly even with a short washing time. The washing time is, from the viewpoint of allowing stains attached to fibers to be easily removed or finishing fibers more softly, preferably 1 minute or more, more preferably 2 minutes or more and further preferably 3 minutes or more, and from the viewpoint of finishing fibers more softly, preferably 1 hour or less, more preferably 30 minutes or less, further preferably 20 minutes or less and furthermore preferably 15 minutes or less.

The method for washing fibers of the present invention is suitable for a method of immersing fibers in a liquid used for refining fibers while feeding the fibers with a roller or the like, and a rotary washing method. The rotary washing method refers to a washing method in which fibers not fixed to a rotating device rotate together with the detergent liquid around the rotation axis. The rotary washing method can be carried out by a rotary type washing machine. Therefore, in the present invention, fibers are preferably washed by using a rotary type washing machine for the viewpoint of finishing the fibers more softly. Specific examples of the rotary type washing machine include a drum type washing machine, a pulsator type washing machine or an agitator type washing machine. As these rotary type washing machines, machines commercially available for household can be used, respectively.

<Embodiments of the present invention>

Embodiments of the present invention will be illustrated as follows. The matters described with respect to the detergent composition for fibers and the method for washing fibers according to the present invention can be appropriately applied to these embodiments.

<1> A detergent composition for fibers containing the following component (A), component (B) and component (C):

component (A): an internal olefin sulfonate having 17 or more and 24 or less carbon atoms,
component (B): a nonionic surfactant, and
component (C): a metal ion chelating agent,

wherein a mass ratio (B)/(A) of component (B) to component (A) is 0 or more and 1.0 or less, the composition contains a nonionic surfactant having an HLB of more than 10.5 [hereinafter referred to as component (B1)] when the composition contains component (B), a content of component (C) is 20% by mass or less, and the composition is a composition for use in washing fibers in water containing a hardness component.

<2> The detergent composition for fibers according to <1>, wherein a content of the internal olefin sulfonate with the sulfonate group at position 5 or higher in component (A) is preferably 60% by mass or less, more preferably 55% by mass or less, further preferably 45% by mass or less, furthermore preferably 40% by mass or less, furthermore preferably 35% by mass or less, furthermore preferably 30% by mass or less, furthermore preferably 25% by mass or less and furthermore preferably 20% by mass or less, and preferably more than 0% by mass and more preferably 5% by mass or more.

<3> The detergent composition for fibers according to <1> or <2>, wherein a mass ratio of the content of an internal olefin sulfonate with the sulfonate group at position 2 or higher and position 4 or lower to a content of the internal olefin sulfonate with the sulfonate group at position 5 or higher and position 9 or lower in component (A), that is, (content of the internal olefin sulfonate with the sulfonate group at position 2 or higher and position 4 or lower)/(content of the internal olefin sulfonate with the sulfonate group at position 5 or higher and position 9 or lower) is preferably 0.5 or more, more preferably 0.8 or more, further preferably 1.0 or more, furthermore preferably 1.5 or more, furthermore preferably 2 or more, furthermore preferably 2.5 or more, furthermore preferably 3 or more, furthermore preferably 4 or more and furthermore preferably 4.5 or more, and preferably 20 or less, more preferably 10 or less, further preferably 8 or less and furthermore preferably 6 or less.

<4> The detergent composition for fibers according to any of <1> to <3>, wherein a content of an olefin sulfonate with the sulfonate group at position 1 in component (A) is preferably 10% by mass or less, more preferably 7% by mass or less, further preferably 5% by mass or less and furthermore preferably 3% by mass or less, and preferably 0.01% by mass or more.

<5> The detergent composition for fibers according to any of <1> to <4>, wherein the HLB of component (B1) is preferably 11 or more, more preferably 12 or more, further preferably 13 or more, furthermore preferably 14 or more, furthermore preferably 15 or more and furthermore preferably 16 or more, and preferably 19 or less.

<6> The detergent composition for fibers according to any of <1> to <5>, wherein component (B1) is a nonionic surfactant represented by the following general formula (b1) :

$$R^1(CO)_mO\text{-}(A^1O)_n\text{-}R^2 \qquad (b1)$$

wherein $R^1$ is an aliphatic hydrocarbon group having 9 or more and 16 or less carbon atoms, $R^2$ is a hydrogen atom or a methyl group, CO is a carbonyl group, m is 0 or 1, $A^1O$ group is one or more groups selected from an ethyleneoxy group and a propyleneoxy group, and n is an average number of added moles and is 6 or more and 50 or less.

<7> The detergent composition for fibers according to <6>, wherein in the general formula (b1), the number of carbon atoms of $R^1$ is 9 or more, preferably 10 or more and more preferably 11 or more, and 16 or less, preferably 15 or less and more preferably 14 or less.

<8> The detergent composition for fibers according to <6> or <7>, wherein the aliphatic hydrocarbon group of $R^1$ is a group selected from an alkyl group and an alkenyl group.

<9> The detergent composition for fibers according to any of <6> to <8>, wherein in the general formula (b1), the $A^1O$ group is a group containing an ethyleneoxy group.

<10> The detergent composition for fibers according to any of <6> to <9>, wherein in the general formula (b1), n is 6 or more, preferably 6.5 or more, more preferably 7 or more, further preferably 8 or more, furthermore preferably 9 or more, furthermore preferably 10 or more and furthermore preferably 12 or more, and 50 or less, preferably 45 or less, more preferably 40 or less, further preferably 35 or less, furthermore preferably 26 or less and furthermore preferably 24 or less.

<11> The detergent composition for fibers according to any of <1> or <10>, wherein component (C) is one or more metal ion chelating agents selected from (C1) a metal ion chelating agent which is an inorganic compound and (C2) a metal ion chelating agent which is an organic compound.

<12> The detergent composition for fibers according to <11>, wherein (C1) the metal ion chelating agent which is an inorganic compound is one or more selected from (C1-1) an alkali metal silicate, (C1-2) an aluminosilicate and (C1-3) a tripolyphosphate.

<13> The detergent composition for fibers according to <11> or <12>, wherein (C2) the metal ion chelating agent which is an organic compound is one or more organic compounds selected from:

(C2-1) a divalent or higher and tetravalent or lower carboxylic acid having 4 or more and 12 or less carbon atoms and containing no amino group, or a salt thereof,
(C2-2) a divalent or higher and tetravalent or lower carboxylic acid having 4 or more and 10 or less carbon atoms and containing an amino group, or a salt thereof, and
(C2-3) a compound having a phosphonate group or a salt thereof in the molecule.

<14> The detergent composition for fibers according to any of <1> to <13>, further containing water.

<15> The detergent composition for fibers according to any of <1> to <14>, wherein the mass ratio (B)/(A) of component (B) to component (A) is 0 or more and 1.0 or less.

<16> The detergent composition for fibers according to any of <1> to <15>, wherein the detergent composition contains component (B), and the content of component (B1) in component (B) is preferably 60% by mass or more, more preferably 70% by mass or more, further preferably 80% by mass or more and furthermore preferably 90% by mass or more, and preferably 100% by mass or less or 100% by mass.

<17> The detergent composition for fibers according to any of <1> to <16>, wherein the mass ratio (B)/(A) of the content of component (B) to the content of component (A) is preferably 0.05 or more, more preferably 0.08 or more, further preferably 0.1 or more, furthermore preferably 0.15 or more and furthermore preferably 0.2 or more, and 1.0 or less, preferably 0.9 or less, more preferably 0.8 or less, further preferably 0.7 or less, furthermore preferably 0.6 or less, furthermore preferably 0.5 or less, furthermore preferably 0.45 or less, furthermore preferably 0.40 or less, furthermore preferably 0.35 or less and furthermore preferably 0.30 or less.

<18> The detergent composition for fibers according to any of <1> to <17>, wherein the content of component (C) is preferably 18% by mass or less, more preferably 15% by mass or less, further preferably 10% by mass or less, furthermore preferably 7% by mass or less, furthermore preferably 5% by mass or less, furthermore preferably 4% by mass or less, furthermore preferably 3% by mass or less, furthermore preferably 1% by mass or less and furthermore preferably 0.5% by mass or less, and preferably more than 0% by mass and more preferably 0.5% by mass or more.

<19> The detergent composition for fibers according to any of <1> to <18>, wherein the content of component (C) is 0% by mass, i.e., the detergent composition has no component (C).

<20> The detergent composition for fibers according to any of <1> to <19>, wherein a mass ratio (C)/(A) of the content of component (C) to the content of component (A) is preferably 2 or less, more preferably 1.5 or less, further preferably 1 or less, furthermore preferably 0.8 or less, furthermore preferably 0.7 or less, furthermore preferably 0.6 or less and furthermore preferably of 0.5 or less, and 0 or more or 0.

<21> The detergent composition for fibers according to any of <1> to <20>, containing (D) a surfactant other than component (A) [hereinafter referred to as component (D)].

<22> The detergent composition for fibers according to <21>, wherein component (D) is one or more surfactants selected from anionic surfactants of the following component (d1), component (d2), component (d3) and component (d4):

component (d1): alkyl or alkenyl sulfate,
component (d2): polyoxyalkylene alkyl ether sulfate or polyoxyalkylene alkenyl ether sulfate,
component (d3): an anionic surfactant having a sulfonate group (except for component (A)), and
component (d4): a fatty acid or a salt thereof,
and a nonionic surfactant of the following component (d5) :
component (d5): a nonionic surfactant having a hydroxy group or polyoxyalkylene group.

<23> The detergent composition for fibers according to <22>, wherein the detergent composition contains one or more anionic surfactants selected from component (d1), component (d2), component (d3) and component (d4) as component (D), and the content of component (A) in the total mass of component (A) and components (d1) to (d4) is preferably 50% by mass or more, more preferably 60% by mass or more, furthermore preferably 70% by mass or more, furthermore preferably 80% by mass or more and furthermore preferably 90% by mass or more, and 100% by mass or less.

<24> The detergent composition for fibers according to any of <1> to <23>, wherein the fibers are fibers containing cotton.

<25> The detergent composition for fibers according to any one of <1> to <24>, wherein the fibers are textile products.

<26> A method for washing fibers with a detergent liquid containing the following component (A) and water, wherein the hardness of the water in the detergent liquid is more than 0°dH:

component (A): an internal olefin sulfonate having 17 or more and 24 or less carbon atoms.

<27> The method for washing fibers according to <26>, wherein the detergent liquid is obtained by using the detergent composition for fibers according to any of <1> to <25>.

<28> The method for washing fibers according to <26> or <27>, wherein the fibers are fibers containing cotton.

<29> The method for washing fibers according to any of <26> to <28>, wherein the fibers are textile products.

<30> The method for washing fibers according to any of <26> to <29>, wherein a mass ratio (C)/(A) of the content of component (C) to the content of component (A) in the detergent liquid is preferably 2 or less, more preferably 1.5 or less, further preferably 1 or less, furthermore preferably 0.8 or less, furthermore preferably 0.7 or less, furthermore preferably 0.6 or less and furthermore preferably of 0.5 or less, and 0 or more or 0.

Examples

<Components to be blended>

In Examples and Comparative Examples, the following components were used.

Synthesis of [component (A)] and [component (A')]

Internal olefins A to E which are raw materials of component (A) and component (A') (a comparative component of component (A)) were synthesized as follows.

Synthesis of internal olefins A to C having 18 carbon atoms (Production Examples A to C)

7000 g (25.9 mol) of 1-octadecanol (product name: KALCOL 8098, manufactured by Kao Corporation) and 700 g of γ-alumina (Strem Chemicals, Inc.) as a solid acid catalyst were introduced into a flask equipped with a stirring device, and allowed to react at 280°C with stirring for a different reaction time for each of Production Examples A to C while circulating nitrogen (7000 mL/min) inside the flask. The resulting crude internal olefin was transferred to a distillation flask and subjected to distillation at 148 to 158°C/0.5 mmHg to obtain each of internal olefins A to C having 18 carbon atoms at an olefin purity of 100%. The double bond distribution of each of the obtained internal olefins is shown in Table 1.

Synthesis of internal olefin D having 16 carbon atoms (Production Example D)

An internal olefin obtained by using the method described in Production Example C of JP-A 2014-76988 was used. The double bond distribution of the obtained internal olefin is shown in Table 1.

[Table 1]

|  |  | Internal olefin | | | |
|---|---|---|---|---|---|
|  |  | A | B | C | D |
| Number of carbon atoms of hydrocarbon group |  | 18 | 18 | 18 | 16 |
| Composition in olefin (% by mass) | Linear olefin | 99.8 | 99.3 | 99.0 | 99.5 |
|  | Branched olefin | 0.2 | 0.7 | 1.0 | 0.5 |
| Distribution of double bond in linear olefin (% by mass) | Position 1 | 1.6 | 0.9 | 0.3 | 0.9 |
|  | Position 2 | 41.7 | 25.0 | 13.3 | 29.8 |
|  | Position 3 | 29.3 | 21.9 | 12.6 | 24.4 |
|  | Position 4 | 15.7 | 19.0 | 13.9 | 18.4 |
|  | Position 5 | 6.3 | 13.6 | 14.8 | 13.0 |
|  | Position 6 | 3.9 | 8.6 | 13.7 | 7.1 |
|  | Position 7 | 1.1 | 5.6 | 12.6 | 3.2 |
|  | Position 8 | 0.2 | 2.7 | 9.4 | 3.2 |
|  | Position 9 | 0.2 | 2.7 | 9.4 | 0.0 |
|  | Total of positions 5 to 9 | 11.7 | 33.2 | 59.9 | 26.5 |

The double bond distribution of each of the internal olefins was measured by gas chromatography (hereinafter abbreviated as GC). Specifically, the internal olefin was reacted with dimethyl disulfide to form its dithiolated derivative, and then each component was subjected to separation by GC. The double bond distribution of internal olefin was determined from each of the resulting peak areas. For the olefins having 18 carbon atoms, the internal olefin having a double bond at position 8 and the internal olefin having a double bond at position 9 cannot be distinguished from each other in structure but distinguished when they are sulfonated. Therefore, the value obtained by dividing the amount of the internal olefin having a double bond at position 8 by 2 is conveniently shown in each of the columns for positions 8 and 9. Similarly, for the olefin having 16 carbon atoms, the value obtained by dividing the amount of the internal olefin having a double bond at position 7 by 2 is conveniently shown in each of the columns for positions 7 and 8.

The devices and the analysis conditions used for the measurement are as follows: a GC system: "HP6890" (manufactured by Hewlett-Packard Company); a column: "Ultra-Alloy-1 HT Capillary Column" (30 m × 250 μm × 0.15 μm, manufactured by Frontier Laboratories, Ltd.); a detector (hydrogen flame ionization detector (FID)); injection temperature: 300°C; detector temperature: 350°C; and He flow rate: 4.6 mL/min.

Synthesis of components (a-1) to (a-3) and component (a'-1) (Production Examples 1 to 4)

Each of internal olefins A to D was subjected to sulfonation reaction by passing sulfur trioxide gas therethrough using a thin film-type sulfonation reactor equipped with an external jacket while passing cooling water at 20°C through the external jacket. The molar ratio of $SO_3$/the internal olefin during the sulfonation reaction was set at 1.09. The resulting sulfonated product was added to an alkaline aqueous solution which had been prepared using sodium hydroxide in an amount of 1.5 molar times the theoretical acid value, and the mixture was neutralized at 30°C for 1 hour while being stirred. The neutralized product was hydrolyzed by being heated in an autoclave at 160°C for 1 hour to obtain a crude product of each sodium internal olefin sulfonate. 300 g of the crude product was transferred to a separating funnel, 300 mL of ethanol was added thereto and petroleum ether in an amount of 300 mL per time was then added thereto to extract and remove oil-soluble impurities. At this time, inorganic compounds (mainly including sodium sulfate decahydrate) which precipitated at the oil/water interface by the addition of ethanol was also separated and removed from the aqueous phase by oil-water separation operation. This extraction and removal operation was carried out three times. The aqueous phase was evaporated to dryness to obtain each of the following sodium internal olefin sulfonates. The mass ratio of the olefin form (sodium olefin sulfonate)/the hydroxy form (sodium hydroxyalkane sulfonate) of each component is as follows.

[Component (A)]

(a-1): a sodium internal olefin sulfonate obtained from internal olefin A
The mass ratio of the olefin form (sodium olefin sulfonate)/the hydroxy form (sodium hydroxyalkane sulfonate) in the sodium internal olefin sulfonate is 18/82.
(a-2): a sodium internal olefin sulfonate obtained from internal olefin B
The mass ratio of the olefin form (sodium olefin sulfonate)/the hydroxy form (sodium hydroxyalkane sulfonate) in the sodium internal olefin sulfonate is 17/83.
(a-3): a sodium internal olefin sulfonate obtained from internal olefin C
The mass ratio of the olefin form (sodium olefin sulfonate)/the hydroxy form (sodium hydroxyalkane sulfonate) in the sodium internal olefin sulfonate is 17/83.

[Component (A')] (a comparative component of Component (A))

(a'-1): a sodium internal olefin sulfonate obtained from internal olefin D
The mass ratio of the olefin form (sodium olefin sulfonate)/the hydroxy form (sodium hydroxyalkane sulfonate) in the sodium internal olefin sulfonate is 16/84.
The mass ratio of the olefin form/the hydroxy form of each component was measured by high performance liquid chromatography/mass spectrometer (HPLC-MS). Specifically, identification was carried out by separating the hydroxy form and the olefin form from each other by high performance liquid chromatography (HPLC) and subjecting each of them to mass spectrometer (MS). Each percentage was determined from the resulting HPLC-MS peak area.
The devices and the analysis conditions used for the measurement are as follows: an HPLC device: "Agilent Technology 1100" (manufactured by Agilent Technologies); a column: "L-column ODS" (4.6 × 150 mm, manufactured by Chemicals Evaluation and Research Institute, Japan); sample preparation (1000 times diluted with methanol); eluent A (10 mM ammonium acetate-added water), eluent B (10 mM ammonium acetate-added methanol); gradient (0 min (A/B = 30/70%) → 10 minutes (30/70%) → 55 minutes (0/100%) → 65 minutes (0/100%) → 66 minutes (30/70%) → 75 minutes (30/70%); an MS device: "Agilent Technology 1100MSSL (G1946D)" (manufactured by Agilent Technologies); and MS detection (negative ion detection, m/z: 60 - 1600, UV 240 nm).
The percentage of the content of the internal olefin sulfonate having the sulfonate group attached thereto of each component was measured by high performance liquid chromatography/mass spectrometer (HPLC-MS). Specifically, identification was carried out by separating the hydroxy form having a sulfonate group attached thereto by high performance liquid chromatography (HPLC) and subjecting it to mass spectrometer (MS). Each percentage was determined from the resulting HPLC-MS peak area. In the present specification, each percentage determined from the peak area was calculated as percentage by mass.
The devices and the analysis conditions used for the measurement are as follows: an HPLC device: "LC-20ASXR" (manufactured by Shimadzu Corporation); a column: "ODS Hypersil (R)" (4.6 × 250 mm, particle size: 3 μm, manufactured by Thermo Fisher Scientific K.K.); sample preparation (1000 times diluted with methanol); eluent A (10 mM ammonium acetate-added water); eluent B (a 10 mM ammonium acetate-added methacrylonitrile/water = 95/5 (v/v) solution); gradient (0 minute (A/B = 60/40) → 15.1 to 20 minutes (30/70) → 20.1 to 30 minutes (60/40); an MS device "LCMS-2020" (manufactured by Shimadzu Corporation); ESI detection (negative ion detection, m/z: 349.15 (component (A) having 18 carbon atoms), 321.10 (component (A') having 16 carbon atoms); column temperature (40°C); flow rate (0.5 mL/min); and injection volume (5 μL).

The distribution of the positions of the carbon through which each of sulfonate groups of (a-1), (a-2), (a-3) and (a'-1) obtained is attached is shown in Table 2.

[Table 2]

|  |  | Internal olefin sulfonate | | | |
|---|---|---|---|---|---|
|  |  | (a-1) | (a-2) | (a-3) | (a'-1) |
| Number of carbon atoms of hydrocarbon group | | 18 | 18 | 18 | 16 |
| Distribution of sulfonate group (% by mass) | Position 1 | 1.7 | 1.4 | 0.6 | 1.5 |
|  | Position 2 | 31.5 | 22.1 | 12.8 | 24.1 |
|  | Position 3 | 25.1 | 17.3 | 10.7 | 19.9 |
|  | Position 4 | 24.7 | 21.8 | 16.6 | 24.6 |
|  | Position 5 | 10.2 | 13.5 | 15.2 | 14.1 |
|  | Positions 6 to 9 | 6.8 | 23.9 | 44.1 | 15.8 |
|  | Total | 100.0 | 100.0 | 100.0 | 100.0 |
|  | Total of positions 2 to 4 | 81.3 | 61.2 | 40.1 | 68.6 |
|  | Total of positions 5 to 9 | 17.0 | 37.4 | 59.3 | 29.9 |
|  | (Total of positions 2 to 4)/ (Total of positions 5 to 9) (mass ratio) | 4.8 | 1.6 | 0.68 | 2.3 |

[Component (B)]

(b-1): a polyoxyethylene lauryl ether (the average number of moles of added oxyethylene group: 10 moles; HLB = 14.0; a compound of the general formula (b1) wherein $R^1$ is a lauryl group, m is 0, $A^1O$ is an ethyleneoxy group, n is 10, and $R^2$ is a hydrogen atom)

(b-2): a polyoxyethylene lauryl ether (the average number of moles of oxyethylene group: 21 moles; HLB = 16.6; a compound of the general formula (b1) wherein $R^1$ is a lauryl group, m is 0, $A^1O$ is an ethyleneoxy group, n is 21, and $R^2$ is a hydrogen atom)

(b-3): a polyoxyalkylene lauryl ether (a compound obtained by adding an average of 9 moles of an ethyleneoxy group per mole of lauryl alcohol, then adding an average of 2 moles of a propyleneoxy group per mole of lauryl alcohol and then adding an average of 9 moles of an ethyleneoxy group per mole of lauryl alcohol; HLB = 14.5; a compound of the general formula (b1) wherein $R^1$ is a lauryl group, m is 0, $A^1O$ is an ethyleneoxy group and a propyleneoxy group, n is 20, and $R^2$ is a hydrogen atom)

(b-4): a polyoxyethylene alkyl ether (the average number of moles of added oxyethylene group: 6 moles; HLB = 12.1; a compound of the general formula (b1) wherein $R^1$ is a mixed alkyl group of a lauryl group and a myristyl group, the ratio of the mass of the lauryl group to the mass of the myristyl group (lauryl group/myristyl group) = 9/1, m is 0, $A^1O$ is an ethyleneoxy group, n is 6, and $R^2$ is a hydrogen atom)

(b-5): a polyoxyethylene alkyl ether (the average number of moles of added oxyethylene group: 5 moles; HLB = 10.7; a compound of the general formula (b1) wherein $R^1$ is a mixed alkyl group of a lauryl group and a myristyl group, the ratio of the mass of the lauryl group to the mass of the myristyl group (lauryl group/myristyl group) = 9/1, m is 0, $A^1O$ is an ethyleneoxy group, n is 5, and $R^2$ is a hydrogen atom)

[Component (B')] (a comparative component of component (B))

(b-1): a polyoxyethylene lauryl ether (the average number of moles of added oxyethylene group: 3 moles; HLB = 8.3; a compound of the general formula (b1) wherein $R^1$ is a lauryl group, m is 0, $A^1O$ is an ethyleneoxy group, n is 3, and $R^2$ is a hydrogen atom)

[Component (C)]

(c1-2-1): zeolite (Zeolite A, manufactured by Zeobuilder Co., Ltd.)
(c1-3-1): sodium tripolyphosphate (manufactured by Thai polyphosphate & Chemicals Co., Ltd.)
(c2-1-1): tetrasodium ethylenediaminetetraacetate (manufactured by Wako Pure Chemical Industries, Ltd.)

[Optional component]

[Component (D)]

(d-1): a sodium alkylbenzene sulfonate (alkyl composition: C10/C11/C12/C13 = 11/29/34/26 (mass ratio); mass average number of carbon atoms = 17.75)

[Component (E)]

(e-1): sodium carbonate

[Water]

Ion-exchanged water

<Preparation of detergent compositions for fibers>

Detergent compositions for fibers shown in Table 3 (Tables 3a and 3b), Table 4, and Table 5 were prepared using the above-mentioned components to be blended, and were evaluated for the following items. The results are shown in Tables 3 to 5.

Specifically, the method for preparing the detergent compositions for fibers shown in Tables 3 to 5 was as follows. A Teflon (R) stirrer piece having a length of 5 cm was placed in a 200 mL glass beaker and its mass was measured. Next, 80 g of ion-exchanged water at 20°C, either component (A) or component (D) and either component (B) or component (B') were introduced thereinto, component (C) was then introduced thereinto when it was used, and the beaker was sealed at its top side with Saran wrap (R). The beaker containing the contents was placed in a water bath at 60°C placed on a magnetic stirrer, and stirred at 100 r/min for 30 minutes at a water temperature range in the water bath of 60 ± 2°C. Next, the water in the water bath was replaced with tap water at 5°C and cooled until the temperature of the composition in the beaker was 20°C. Next, Saran Wrap (R) was removed, ion-exchanged water was added so that the mass of the contents was 100 g and stirred again at 100 r/min for 30 seconds to obtain each of the detergent compositions for fibers shown in Tables 3 to 5.

The detergent compositions for fibers containing zeolite as component (C) were thoroughly stirred before weighing them, and weighed with zeolite uniformly dispersed.

<Evaluation method of softness>

(1) Pretreatment of fibers to be evaluated

(1-1) Pretreatment of knitted cotton

1.7 kg of knitted cotton (un-mercerized knitted cotton (not mercerized one), cotton 100%, manufactured by Shikisensha Co., Ltd.) was previously washed cumulatively twice with a standard course of a fully automatic washing machine (NA-F702 P manufactured by Matsushita Electric Industrial Co., Ltd.) (4.7 g of Emulgen 108 (manufactured by Kao Corporation) at washing; the amount of water: 47 L; washing for 9 minutes, rinsing twice and spin-drying for 3 minutes) followed by cumulatively washing three times with water only (the amount of water: 47 L; washing for 9 minutes, rinsing twice and spin-drying for 3 minutes), and dried under an environment of 23°C and 45% RH for 24 hours.

(1-2) Pretreatment of polyester cloth

1.6 kg of a commercially available polyester jersey (available from Dyeing Test Material Co., Ltd. Tanigashira Shoten; polyester 100%, 30 cm × 30 cm cut piece) was previously subjected to a washing process using a nonionic surfactant (ethylene oxide adduct of lauryl alcohol (average number of added moles: 8) with an automatic washing machine (NW-6CY, manufactured by Hitachi, Ltd.) 5 times repeatedly (the amount of nonionic surfactant used: 4.5 g; a standard course; the amount of water: 45 L; water temperature: 20°C; washing time: 10 minutes; rinsing with accumulated water: twice). Then, it was dried for 1 day under the conditions of 20°C and 43% RH.

(2) Washing of fibers to be evaluated

6.0 L of municipal water (3.5°dH, calculated by the method for measuring a hardness of water described above, 20°C) was poured into an electric bucket type washing machine (model number "N-BK 2", manufactured by Matsushita Electric Industrial Co., Ltd.), 12 g of each of the detergent compositions for fibers shown in Tables 3 to 5 g was introduced thereinto, the mixture was stirred for 1 minute. Then, four cut pieces of the knitted cotton (approximately 140 g) or 170 g of the polyester jersey which had been pretreated by the above method were introduced thereinto and washed for 10 minutes. After washing, spin-drying was carried out for 1 minute using a two-compartment washing machine (model number: "PS-H35L", manufactured by Hitachi, Ltd.). Next, 6.0 L of municipal water was poured into the bucket type washing machine, and the knitted cotton or polyester jersey after spin-dried with the two-compartment washing machine manufactured by Hitachi, Ltd. was introduced thereinto and rinsed for 3 minutes. The same spin-drying was then carried out for 1 minute using the two-compartment washing machine. After this rinsing was carried out twice in total, the knitted cotton or polyester jersey was allowed to stand for 12 hours under the conditions of 20°C and 43% RH to dry it. For Comparative Example 8 in Table 4, washing was carried out using ion-exchanged water (0°dH, calculated by the method for measuring hardness of water described above, 20°C) in place of municipal water.

(3) Evaluation of softness

The softness of the knitted cotton or polyester jersey, which had been washed with detergent composition for fibers as shown in Tables 3 to 5, rinsed and dried, was scored according to the following criteria by six experts skilled in the texture evaluation of fibers, and the average value of the scores of six experts was calculated. The results are shown in Tables 3 to 5.

-1 ⋯ It was not finished more softly than the knitted cotton or polyester jersey treated with the composition of Comparative Example 1.
0 ⋯ It was finished as softly as the knitted cotton or polyester jersey treated with the composition of Comparative Example 1.
1 ⋯ It was finished somewhat more softly than the knitted cotton or polyester jersey treated with the composition of Comparative Example 1.
2 ⋯ It was finished more softly than the knitted cotton or polyester jersey treated with the composition of Comparative Example 1.
3 ⋯ It was finished much more softly than the knitted cotton or polyester jersey treated with the composition of Comparative Example 1.

Table 4 shows the evaluation using the composition of Comparative Example 9 as a reference, and Table 5 shows the evaluation of the knitted cotton washed using the composition of Comparative Example 10 as a reference and the evaluation of the polyester jersey washed using the composition of Comparative Example 11 as a reference.

<Evaluation method of detergent property>

(1) Preparation of the model artificially sebum-stained cloth

A model artificially sebum-stained cloth was prepared by applying a model artificially sebum-staining liquid of the following composition to a cloth (Cotton 2003 (manufactured by Tanigashira Shoten)). The application of the model artificially sebum-staining liquid to the cloth was carried out by printing the artificially staining liquid on the cloth using a gravure roll coater. The process for preparing the model artificially sebum-staining liquid by applying the model artificially sebum-staining liquid to the cloth was carried out with a cell capacity of the gravure roll of 58 cm$^3$/m$^2$, a coating speed of 1.0 m/min, a drying temperature of 100°C and a drying time of 1 minute. The cloth used was Cotton 2003 (manufactured by Tanigashira Shoten).

**[0142]** * The composition of the model artificially sebum-staining liquid: lauric acid: 0.4% by mass, myristic acid: 3.1% by mass, pentadecanoic acid: 2.3% by mass, palmitic acid: 6.2% by mass, heptadecanoic acid: 0.4% by mass, stearic acid: 1.6 % by mass, oleic acid: 7.8% by mass, triolein: 13.0% by mass, n-hexadecyl palmitate: 2.2% by mass, squalene: 6.5% by mass, egg white lecithin liquid crystal product: 1.9% by mass, Kanuma red clay: 8.1% by mass, carbon black:

0.01% by mass and water: balance (total 100% by mass).

(2) Evaluation of detergency

Five cut pieces of the model artificially sebum-stained cloth (6 cm × 6 cm) as prepared above were washed at 85 rpm in Terg-O-Tometer (MS-8212, manufactured by Ueshima Seisakusho Co., Ltd.) for five minutes or ten minutes. For washing conditions, washing was carried out at a water temperature of 20°C by pouring municipal water (3.5°dH, calculated by the method for measuring the hardness of water described above, 20°C) so that each of the concentrations of the detergent compositions for fibers shown in Tables 3 to 5 was 0.033% by mass. After washing, rinsing with municipal water (20°C) was carried out for 3 minutes. For Comparative Example 8 in Table 4, washing was carried out using ion-exchanged water (0°dH, calculated by the method for measuring hardness of water described above, 20°C) in place of municipal water.
The washing percentage (%) was measured by the following method, and the average value of washing percentages of the five cut pieces was determined. The results are shown in Tables 3 and 4.
The reflectance at 550 nm of each of the original cloth before staining and the clothes before and after washing was measured with a differential colorimeter (Z-300A, manufactured by Nippon Denshoku Industries Co., Ltd.), and the washing percentage (%) was determined by the following formula (the values in the Tables are average values of the washing percentages for 5 cut pieces).

```
Washing percentage (%) = 100 × [(reflectance after

washing - reflectance before washing)/(reflectance of

original cloth - reflectance before washing)]
```

| | | | | Example | | | | | | | | | | | | |
|---|---|---|---|---|---|---|---|---|---|---|---|---|---|---|---|---|
| | | | | 1 | 2 | 3 | 4 | 5 | 6 | 7 | 8 | 9 | 10 | 11 | 12 | 13 |
| Detergent composition for fibers | Content (% by mass) | Component (A) | (a-1) | | | | | | | | | | | 10 | | |
| | | | (a-2) | 5 | 6 | 7 | 7.5 | 8 | 9 | 9.8 | 8 | 8 | 9 | | 10 | |
| | | | (a-3) | | | | | | | | | | | | | 10 |
| | | Component (A') | (a'-1) | | | | | | | | | | | | | |
| | | Component (B) | (b-1) | 5 | 4 | 3 | 2.5 | 2 | 1 | 0.2 | | | | | | |
| | | | (b-2) | | | | | | | | | | 1 | | | |
| | | | (b-3) | | | | | | | | | | | | | |
| | | | (b-4) | | | | | | | | 2 | | | | | |
| | | | (b-5) | | | | | | | | | 2 | | | | |
| | | Component (B') | (b'-1) | | | | | | | | | | | | | |
| | | Component (C) | (c 1 -2-1) | | | | | | | | | | | | | |
| | | | (c 1 -3-1) | | | | | | | | | | | | | |
| | | | (c2-1-1) | | | | | | | | | | | | | |
| | | Component (D) | (d-1) | | | | | | | | | | | | | |
| | Ion-exchanged water | | | Balance | Balance | Balance | Balance | Balance | Balance | Balance | Balance | Balance | Balance | Balance | Balance | Balance |
| | Total | | | 100 | 100 | 100 | 100 | 100 | 100 | 100 | 100 | 100 | 100 | 100 | 100 | 100 |
| | (B)/(A) (mass ratio) | | | 1.00 | 0.67 | 0.43 | 0.33 | 0.25 | 0.11 | 0.020 | 0.25 | 0.25 | 0.11 | 0 | 0 | 0 |
| Evaluation results | Feeling performance (knitted cotton) | | | 1.2 | 1.5 | 2.0 | 2.2 | 2.4 | 2.5 | 2.9 | 2.2 | 1.8 | 2.5 | 3.0 | 2.9 | 2.7 |
| | Detergent performance (%) | | | 38 | 38 | 40 | 40 | 40 | 39 | 35 | 38 | 26 | 39 | 35 | 33 | 25 |

[Table 3b]

| | | Example | | | | | | | | Comparative Example | | | | | | |
|---|---|---|---|---|---|---|---|---|---|---|---|---|---|---|---|---|
| | | 14 | 15 | 16 | 17 | 18 | 19 | 20 | 21 | 1 | 2 | 3 | 4 | 5 | 6 | 7 |
| Detergent composition for fibers — Content (% by mass) — Component (A) | (a-1) | 9.5 | | | | | | | | | | | | | | |
| | (a-2) | | 9.5 | | 9 | 8 | 8 | 6 | 10 | | 5 | 3 | 10 | 10 | 10 | |
| | (a-3) | | | 9.5 | | | | | | | | | | | | |
| Component (A') | (a'-1) | | | | | | | | | | | | | | | 10 |
| Component (B) | (b-1) | 0.5 | 0.5 | 0.5 | | | | | | 5 | | | | | | |
| | (b-2) | | | | | | | | | | | 7 | | | | |
| | (b-3) | | | | 1 | 2 | | | | | | | | | | |
| | (b-4) | | | | | | | | | | | | | | | |
| | (b-5) | | | | | | | | | | | | | | | |
| Component (B') | (b'-1) | | | | | | | | 5 | | 5 | | | | | |
| Component (C) | (c1-2-1) | | | | | | | | | | | | 25 | | | |
| | (c1-3-1) | | | | | | | | | | | | | 25 | | |
| | (c2-1-1) | | | | | | | | | | | | | | 25 | |
| Component (D) | (d-1) | | | | | | | | | 5 | | | | | | |
| | Ion-exchanged water | Balance | Balance | Balance | Balance | Balance | Balance | Balance | Balance | Balance | Balance | Balance | Balance | Balance | Balance | Balance |
| | Total | 100 | 100 | 100 | 100 | 100 | 100 | 100 | 100 | 100 | 100 | 100 | 100 | 100 | 100 | 100 |
| (B)/(A) (mass ratio) | | 0.053 | 0.053 | 0.053 | 0.11 | 0.25 | 0 | 0 | 0 | - | - | 2.33 | 0 | 0 | 0 | 0 |
| Evaluation results | Feeling performance (knitted cotton) | 3.0 | 2.9 | 2.7 | 2.2 | 2.0 | 2.2 | 1.8 | 2.5 | Reference | 0.2 | -1 | 0.5 | 0.5 | -0.5 | 0 |
| | Detergent performance (%) | 39 | 37 | 30 | 34 | 36 | 31 | 30 | 33 | 41 | 33 | 44 | 38 | 39 | 34 | 34 |

(Discussion)

Table 3 shows that fibers can be finished softly by each of the detergent compositions for fibers containing component (A) having a particular number of carbon atoms according to the present invention. It is also shown that stains attached to the fibers can also be washed off by each of the detergent compositions for fibers of the present invention.

[Table 4]

| | | | | Example | Comparative Example | |
|---|---|---|---|---|---|---|
| | | | | 22 | 8 | 9 |
| Detergent composition for fibers | Content (% by mass) | Component (A) | (a-2) | 7 | 7 | |
| | | Component (B) | (b-1) | 3 | 3 | 5 |
| | | Component (D) | (d-1) | | | 5 |
| | | Ion-exchanged water | | Balance | Balance | Balance |
| | | Total | | 100 | 100 | 100 |
| | (B)/(A) (mass ratio) | | | 0.43 | 0.43 | - |
| Hardness of water used for washing (°dH) | | | | 3.5 | 0 | 3.5 |
| Evaluation results | Feeling performance (knitted cotton) | | | 2.9 | -1 | 0 (Reference) |
| | detergent property | | | 38 | 40 | 41 |

(Discussion)

Table 4 shows that when using ion-exchanged water instead of municipal water, the fibers evaluated were not finished softly by washing using ion-exchanged water with a hardness of 0°dH. The detergent composition for fibers of the present invention is suitable for washing with water containing a hardness component.

[Table 5]

| | | | | Example | Comparative Example | Example | Comparative Example |
|---|---|---|---|---|---|---|---|
| | | | | 23 | 10 | 24 | 11 |
| Detergent composition for fibers | Content (% by mass) | Component (A) | (a-2) | 10 | | 10 | |
| | | Component (D) | (d-1) | | 10 | | 10 |
| | | Component (E) | (e-1) | 1 | 1 | 1 | 1 |
| | | Water | | Balance | Balance | Balance | Balance |
| | | Total | | 100 | 100 | 100 | 100 |
| | Content of component (A) in surfactant (mass%) | | | 100 | 0 | 100 | 0 |
| | (B)/(A) (mass ratio) | | | 0 | 0 | 0 | 0 |
| Evaluation results | Type of washed fiber | | | Knitted cotton | Knitted cotton | Polyester | Polyester |
| | Feeling performance | | | 2.2 | 0 (Reference) | 1.0 | 0 (Reference) |

(Discussion)

Table 5 shows that when the fibers evaluated were knitted cotton, the difference between the evaluation scores of softness of Example 23 and Comparative Example 10 was 2.2. On the other hand, when the fibers evaluated were polyester, the difference between the evaluation scores of softness of Example 24 and Comparative Example 11 was 1.0. The detergent composition for fibers of the present invention is a composition excellent in the performance for finishing fibers more softly in washing cotton fibers.

**Claims**

1.  A detergent composition for fibers comprising the following component (A), component (B) and component (C):

    component (A): an internal olefin sulfonate having 17 or more and 24 or less carbon atoms,
    component (B): a nonionic surfactant, and
    component (C): a metal ion chelating agent,

    wherein a mass ratio (B)/(A) of the component (B) to the component (A) is 0 or more and 1.0 or less, the composition contains a nonionic surfactant having an HLB of more than 10.5 [hereinafter referred to as a component (B1)] when the composition contains the component (B), a content of the component (C) is 20% by mass or less, and the composition is a composition for use in washing fibers in water containing a hardness component.

2.  The detergent composition for fibers according to claim 1, wherein the component (B1) is a nonionic surfactant represented by the following general formula (b1) :

    $$R^1(CO)_mO\text{-}(A^1O)_n\text{-}R^2 \qquad (b1)$$

    wherein $R^1$ is an aliphatic hydrocarbon group having 9 or more and 16 or less carbon atoms, $R^2$ is a hydrogen atom or a methyl group, CO is a carbonyl group, m is 0 or 1, $A^1O$ group is one or more groups selected from an ethyleneoxy group and a propyleneoxy group, and n is an average number of added moles and is 6 or more and 50 or less.

3.  The detergent composition for fibers according to claim 1 or 2, further comprising water.

4.  The detergent composition for fibers according to any one of claims 1 to 3, wherein the mass ratio (B)/(A) of the component (B) to the component (A) is 0 or more and 0.5 or less.

5.  The detergent composition for fibers according to any one of claims 1 to 4, wherein the fibers are fibers containing cotton.

6.  The detergent composition for fibers according to any one of claims 1 to 5, wherein the fibers are textile products.

7.  A method for washing fibers with a detergent liquid comprising the following component (A) and water, wherein a hardness of the detergent liquid is more than 0°dH:
    component (A): an internal olefin sulfonate having 17 or more and 24 or less carbon atoms.

8.  The method for washing fibers according to claim 7, wherein the detergent liquid is obtained by using the detergent composition for fibers according to any one of claims 1 to 4.

9.  The method for washing fibers according to claim 7 or 8, wherein the fibers are fibers containing cotton.

10. The method for washing fibers according to any one of claims 7 to 9, wherein the fibers are textile products.

## INTERNATIONAL SEARCH REPORT

| | International application No. |
|---|---|
| | PCT/JP2017/020060 |

**A. CLASSIFICATION OF SUBJECT MATTER**

*C11D1/14*(2006.01)i, *C07C43/10*(2006.01)i, *C07C43/11*(2006.01)i, *C07C229/16*(2006.01)i, *C07C309/20*(2006.01)i, *C11D1/72*(2006.01)i, *C11D1/74*(2006.01)i, *D06L1/12*(2006.01)i

According to International Patent Classification (IPC) or to both national classification and IPC

**B. FIELDS SEARCHED**

Minimum documentation searched (classification system followed by classification symbols)
C11D1/14, C07C43/10, C07C43/11, C07C229/16, C07C309/20, C11D1/72, C11D1/74, D06L1/12

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2017 |
| Kokai Jitsuyo Shinan Koho | 1971–2017 | Toroku Jitsuyo Shinan Koho | 1994–2017 |

Electronic data base consulted during the international search (name of data base and, where practicable, search terms used)
JSTPlus/JMEDPlus/JST7580(JDreamIII), Keyword: IRYOYO SENZAI, NAIBU OREFIN (in Japanese) and related terms

**C. DOCUMENTS CONSIDERED TO BE RELEVANT**

| Category* | Citation of document, with indication, where appropriate, of the relevant passages | Relevant to claim No. |
|---|---|---|
| X | JP 60-96692 A (Lion Corp.),<br>30 May 1985 (30.05.1985),<br>claims; page 2, lower left column, line 8 to page 3, upper left column, table 1, samples 2 to 4<br>(Family: none) | 1-10 |
| X | JP 3-126793 A (Unilever N.V.),<br>29 May 1991 (29.05.1991),<br>page 8, upper right column, table 2, examples 9 to 10<br>& GB 2236538 A<br>page 15, table 2, examples 9 to 10 | 1-10 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 26 June 2017 (26.06.17) | 04 July 2017 (04.07.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | |
| | Telephone No. |

Form PCT/ISA/210 (second sheet) (January 2015)

**INTERNATIONAL SEARCH REPORT**

International application No.

PCT/JP2017/020060

C (Continuation).  DOCUMENTS CONSIDERED TO BE RELEVANT

| Category* | Citation of document, with indication, where appropriate, of the relevant passages | Relevant to claim No. |
|---|---|---|
| X | US 5078916 A  (SHELL OIL CO.),<br>07 January 1992 (07.01.1992),<br>claims; column 6, line 45 to column 7, line 10;<br>examples 6 to 7<br>& EP 377261 A2 | 1-3,5-10 |
| X | JP 2003-81935 A  (Lion Corp.),<br>19 March 2003 (19.03.2003),<br>claims; paragraphs [0037] to [0047];<br>prescription example 2<br>(Family: none) | 1-3,5-10 |
| A | JP 60-96693 A  (Lion Corp.),<br>30 May 1985 (30.05.1985),<br>claims; page 3, upper right column, table 1,<br>sample 3<br>(Family: none) | 1-10 |
| A | WO 2014/046175 A1  (Kao Corp.),<br>27 March 2014 (27.03.2014),<br>claims<br>& EP 2899258 A1<br>claims<br>& JP 2014-76988 A        & CN 104603251 A | 1-10 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014076988 A
- EP 377261 A
- JP 2003081935 A
- JP 3126793 A
- JP 6316700 A

**Non-patent literature cited in the description**

- *J. Am. Oil Chem. Soc.,* 1992, vol. 69, 39
- **KUNIEDA et al.** *Journal of Colloid and Interface Science,* September 1985, vol. 107 (1